# EUROPÄISCHE PATENTSCHRIFT

(11) **EP 1 420 853 B1**
(45) Veröffentlichungstag und Bekanntmachung des Hinweises auf die Patenterteilung: **07.04.2010**
(21) Anmeldenummer: 02752932.0
(22) Anmeldetag: 23.08.2002
(51) Int. Cl.: A61M 39/04, A61M 1/00

(54) **MEMBRANE-KANÜLEN-KOMBINATION UND KONNEKTIERUNGSVORRICHTUNG**
MEMBRANE/CANNULA COMBINATION AND CONNECTING DEVICE
COMBINAISON MEMBRANE ET CANULE ET DISPOSITIF DE LIAISON

(30) Priorität: 31.08.2001 DE 10142637; 06.09.2001 DE 20114795 U; 06.09.2001 DE 10143820
(43) Veröffentlichungstag der Anmeldung: 26.05.2004
(73) Patentinhaber: F. Hoffmann-La Roche AG, 4070 Basel (CH); Roche Diagnostics GmbH, 68305 Mannheim (DE)
(72) Erfinder: REINMANN, Andreas, c/o Intec Inst. für Technologietransfer, CH-3014 Bern (CH); HUNN, Marcel, CH-4900 Langenthal (CH)
(74) Vertreter: Wess, Wolfgang
(86) Internationale Anmeldenummer: PCT/CH2002/000460
(87) Internationale Veröffentlichungsnummer: WO 2003/020360

(56) Entgegenhaltungen:
- WO-A-99/34754
- US-A- 5 354 275
- US-A- 5 405 331
- US-A- 5 520 641

## Beschreibung

Die Erfindung betrifft eine Membrane-Kanülen-Kombination und eine Konnektierungsvorrichtüng für biotechnische Anwendungen, vorzugsweise für medizinaltechnische Anwendungen und andere Sterilanwendungen. Besonders bevorzugt findet die Erfindung bei Infusionseinrichtungen, Dialyse-, Perfusions- oder Spüleinrichtungen und messtechnischen Einrichtungen Verwendung.

In der Medizinaltechnik, insbesondere in der Humanmedizin, müssen Fluidführungsleitungen, beispielsweise Katheter, miteinander dicht und steril verbunden werden. Besonders kritisch ist solch eine Verbindung im Falle einer Körperzugangsvorrichtung, die perkutan oder subkutan dauerhaft in einem biologischen Gewebe, insbesondere im menschlichen Körper, implantiert ist. Kann eine Verbindung erst nach der Implantation hergestellt werden oder soll die Verbindung lösbar und wiederherstellbar sein, so steigen die Anforderungen an die sterile Abdichtung einer Verbindungsstelle zwischen den Fluidführungsleitungen.

Körperzugangsvorrichtungen, wie die Erfindung sie vorzugsweise auch betrifft, sind beispielsweise aus der EP 0 867 197, EP 0 867 196 und EP 0 867 198 bekannt. Bei diesen Vorrichtungen wird eine sterile Abdichtung einer lösbaren und wiederholt herstellbaren Verbindung von zwei Fluidleitungen innerhalb eines perkutan implantierten Portkörpers mittels einer Membrane aus einem elastischen Material hergestellt. Die Membrane ist in dem Portkörper angeordnet und weist einen selbstschließenden Durchgang auf. Die eine Fluidleitung ist ein implantierter Katheter, der in den Portkörper geführt ist. Die andere der zu verbindenden Fluidleitungen wird durch eine feste, dünne Kanüle gebildet, die in den Durchgang einführbar ist. Die Membrane umgibt einen Außenmantel der in den Durchgang eingeführten Kanüle rundum dicht. Die Membrane schafft in dem Portkörper eine dichte Verbindung zwischen dem implantierten Katheter und der von außerhalb des Körpers in den Portkörper geführten Kanüle.

Aus den Druckschriften US 5,405,331 A, US 5,520,641 A und US 5,354,275 A sind Septen bekannt, welche zumindest einen Durchgang zum Durchstecken einer Kanüle aufweisen. Bei den Septen wird beim Einführen der Kanüle das Material der Septen elastisch verformt und in einen sich unmittelbar axial an den Durchgang anschließenden Hohlraum gedrückt. Der Durchgang weitet sich beim Einführen der Kanüle auf, während sich der Hohlraum hinsichtlich seines Durchmessers verengt. Durch die Verformung des Hohlraums soll dessen Wand an der Umfangsfläche der Kanüle dichtend anliegen.

Die US 5,405,331 A weist einen bezüglich seines geometrischen Querschnitts nicht näher spezifizierten Durchgang auf. Die US 5,354,275 A weist einen Durchgang mit einem schlitzförmigen Querschnitt auf. Die US 5,520,641 A weist ein Septum auf, mit mehreren Durchgängen, damit die Kanüle beim Einführen in das Septum in einen der Durchgänge eindringen kann.

Es ist eine Aufgabe der Erfindung, die Abdichtung zwischen einer Membrane und einer in die Membrane eingeführten Kanüle zu verbessern.

Diese Aufgabe wird durch den Gegenstand von Anspruch 1 gelöst.

Die Erfindung umfaßt eine Membrane für biologische Anwendungen, insbesondere für medizinaltechnische Anwendungen und innerhalb der Medizinaltechnik vorzugsweise für Anwendungen in der Humanmedizin. Die Membrane ist jedoch für Anwendung in der Biologie im Allgemeinen, beispielsweise in der Tiermedizin, aber auch außerhalb der Medizin, gleichermaßen geeignet. Die Membrane weist ein elastisches Material auf oder ist gänzlich als ein Membrankörper aus einem elastischen Material gebildet. Das elastische Material kann beispielsweise Silikon oder Latex sein. Auch eine Kombination von mehreren unterschiedlichen elastischen Materialien kann zum Einsatz kommen. Durch die Membrane erstreckt sich wenigstens ein Durchgang, vorzugsweise genau ein Durchgang, für eine Kanüle. Die Membrane ist so beschaffen oder derart in einem Gehäuse eingebaut, dass der Durchgangskanal aufgeweitet wird, wenn eine Kanüle mit einem entsprechend großen Querschnitt in den Durchgang eingeführt ist. Das elastische Material der Membrane drückt im Falle einer radialen Aufweitung des Durchgangs elastisch gegen einen Mantel der Kanüle und umspannt den Kanülemantel elastisch derart dicht, dass im Falle einer bevorzugten Verwendung in einem Fluldführungssystem in der Medizinaltechnik durch die Abdichtung die für den Verwendungszweck erforderliche Sterilität gewährleistet ist.

Nach der Erfindung ist die Membrane quer zu dem Durchgang kompressibel. Im Falle einer vorteilhaften engen Einfassung der Membrane über ihre gesamte Oberfläche kann aufgrund der Kompressibilität die elastische Aufweitung des Durchgangs gewährleistet werden. In bevorzugten Ausführungen ist die Membrane derart in einem Gehäuse eng eingefasst, dass die Membrane in der Einfassung bereits quer zu dem Durchgang komprimiert wird, bevor die Kanüle in den Durchgang eingeführt ist. Besonders bevorzugt wird in diesem Zustand der Durchgang durch die Kompressionskräfte geschlossen. Vorzugsweise ist auch dieser Verschluss ausreichend dicht, um die für medizinische Verwendungen erforderliche Sterilität zu gewährleisten.

Vorzugsweise wird die Kompressibilität der Membrane dadurch erzielt, dass in dem elastischen Membranmaterial neben dem Durchgang wenigstens ein Hohlraum ausgebildet, in den hinein die Membrane bei einer Kompression deformiert werden kann. In den bevorzugten Ausführungsbeispielen weist die Membrane in dem elastischen Membranmaterial neben dem Durchgang mehrere Hohlräume auf. Die mehreren Hohlräume erstrecken sich neben dem Durchgang bevorzugterweise durch die Membrane hindurch. Besonders bevorzugt verlaufen sie parallel zu dem Durchgang. Die Hohlräume können, anstatt durch formgebende bzw. gestaltgebende Maßnahmen oder in Kombination mit der Formgebung auch durch die Poren eines elastischen, porösen, aber in seiner Gesamtheit nicht permeablen Materials gebildet werden, d.h. mittels eines porösen Materials, dessen Poren geschlossen sind. Solch ein poröses Material kann auch ein elastisches, nicht poröses Material umgeben, und der Durchgang würde sich durch das nicht poröse Material erstrecken. Schließlich müssen die Hohlräume oder muss der wenigstens eine Hohlraum auch nicht unbedingt innerhalb des elastischen Materials ausgebildet sein, obgleich dies bevorzugt wird. Die Hohlräume oder der wenigstens eine Hohlraum können als Vertiefungen bzw. Vertiefung an einem äußeren Umfang des elastischen Materials ausgebildet sein.

Durch die Ausbildung wenigstens eines Hohlraums neben dem Durchgang kann der Durchgang grundsätzlich eine beliebige Querschnittsform aufweisen. So kann der Querschnitt des Durchgangs in einem unbelasteten Zustand der Membrane, in dem die Membrane frei ist von äußeren Kräften, über die gesamte Länge des Durchgangs enger als ein äußerer Querschnitt der Kanüle sein.

Erfindungsgemäß weist der Durchgang im unbelasteten Zustand der Membran eine gestreckte Querschnittsfläche mit einer langen Hauptachse und einer dazu senkrechten kurzen Hauptachse auf. Der Durchgang kann im unbelasteten Zustand der Membrane durch einen Schlitz gebildet werden. In diesem Falle ist die Länge der kurzen Hauptachse Null. Vorzugsweise weist der Durchgang jedoch im unbelasteten Zustand der Membrane eine von Null verschiedene Querschnittsfläche auf. Die lange Hauptachse und die kurze Hauptachse sind die lange Seite und die kurze Seite des kleinsten Rechtecks, das die Querschnittsfläche des Durchgangs umgibt. Die einzuführende Kanüle weist üblicherweise einen kreisrunden Querschnitt auf. Die Länge der langen Hauptachse des Querschnitts ist erfindungsgemäß größer als ein mittlerer Kanülendurchmesser. Auf diese Weise kann ein Hohlraum, in den das elastische Material der Membrane ausweichen kann von dem Durchgang gebildet werden. Ein zwischen der Kanüle und dem elastischen Material gebildeter Hohlraum kann bereits den wenigstens einen Hohlraum bilden, durch den die Kompressibilität erhalten wird.

Die erfindungsgemäße Querschnittsform verbessert die Dichtheit des Verschlusses des Durchgangs durch radiale Kompression der Membrane sowohl vor als auch nach Einführung der Kanüle.

Der gestreckte Querschnitt d.h. die gestreckte Querschnittsfläche des Durchgangs ist bevorzugterweise oval. Der Querschnitt kann beispielsweise elliptisch sein. Unter einem Oval wird im Sinne der Erfindung auch ein Querschnitt verstanden, der zwischen gerundeten Bereichen gerade Stücke aufweist. Allerdings wird ein Querschnitt mit einem stetig gekrümmten Umfang bevorzugt.

Weist der Durchgang einen gestreckten Querschnitt auf und sind zusätzlich mehrere Hohlräume neben dem Durchgang angeordnet, wie dies einer besonders bevorzugten Ausführung entspricht, so sind diese Hohlräume in der Querschnittsebene vorteilhafterweise achssymmetrisch in bezug auf wenigstens eine der Hauptachsen, vorzugsweise die lange Hauptachse des Durchgangsquerschnitts angeordnet. Besonders bevorzugt sind sie achssymmetrisch in bezug auf beide Hauptachsen angeordnet. In solch einer Ausbildung des Durchgangs und Anordnung von Hohlräumen wird das elastische Membranmaterial im Falle einer Aufweitung des Durchgangs besonders gleichmäßig in die Hohlräume neben dem Durchgang deformiert, und es wird ein besonders gleichmäßiges Anlegen des elastischen Materials an die eingeführte Kanüle erzielt. In bezug auf eine zu dem Querschnitt mit den beiden Hauptachsen senkrechte Längsachse der Membrane sind die Hohlräume vorzugsweise nicht rotationssymmetrisch um diese Längsachse angeordnet. Die Hohlräume sind vorzugsweise zu der kurzen Hauptachse hin konzentriert, und besonders bevorzugt sind auf der langen Hauptachse und vorzugsweise auch in unmittelbarer Nähe der langen Hauptachse keine Hohlräume vorgesehen.

Vorzugsweise ist die Querschnittsfläche des Durchgangs an ihren schmalen Enden nicht kantig, zumindest nicht scharfkantig, sondern gerundet, vorzugsweise gleichmäßig gerundet. Durch diese Form der Querschnittsfläche kann besonders gut sichergestellt werden, dass eine dicke Kanüle mit einem Außendurchmesser von beispielsweise 1 mm ohne Beschädigung in den Durchgang eingeführt werden kann, und es wird dennoch eine gleichmäßige, elastisch dichte Anlage des Membranmaterials an den Kanülenmantel gewährleistet.

Die Membrane findet bevorzugt Verwendung zur Durchleitung eines biologischen oder biologisch wirksamen Fluids, besonders bevorzugt wird sie für eine Konnektierungsvorrichtung zur Herstellung einer Verbindung zwischen zwei Fluidleitungen verwendet. Ein biologisches Fluid kann beispielsweise eine menschliche oder tierische Körperflüssigkeit sein. Ein biologisch wirksames Fluid kann beispielsweise durch eine medizinisch wirksame Substanz gebildet werden oder solch eine Substanz enthalten.

In bevorzugter Ausführung ist die Konnektiervorrichtung eine Körperzugangsvorrichtung mit einem perkutan oder subkutan implantierbaren Portkörper. Ein perkutan implantierter Portkörper ragt aus der Hautoberfläche hervor und ragt bis in die Haut und vorzugsweise bis unter die Haut in das Körpergewebe hinein. Ein subkutan implantierbarer Portkörper wird unter die Haut implantiert und weist üblicherweise eine zur Haut hin gelegene Membrane auf, die mittels einer unter die Haut gestochenen Nadel zum Zwecke der Herstellung einer Fluidverbindung durchstochen werden kann.

Indem in einer besonders bevorzugten Ausführung des Portkörpers oder auch einer anderen Konnektiervorrichtung eine Gehäuseöffnung für den Katheter in einer Vertiefung einer Gehäuseoberfläche ausgebildet und der Katheter im Anschluss an die Gehäuseöffnung in der Vertiefung aufgenommen ist, kann ein gekrümmtes Katheterstück geschützt werden. Ein Krümmen bzw. Abwinkeln des Katheterstücks unmittelbar im Anschluss an die Gehäuseöffnung ist bei implantierten Kathetern üblich, da die Katheter zur Verringerung der am Katheterende bestehenden Infektionsgefahr über eine gewisse Strecke unmittelbar unter der Haut geführt sind und die Haut sozusagen tunnelisiert wird. Auf diese Weise kann der implantierte Katheter beispielsweise im Falle einer bevorzugten Verwendung im Rahmen einer intravenösen Injektion oder Infusion über mehrere 100 mm bis in die betreffende Vene geführt sein. In Folge des abgewinkelten Verlaufs des Katheters unmittelbar am implantierten Portkörper besteht im Bereich der Abwinklung des Katheters eine erhöhte Gefahr der Verstopfung. Die Verstopfungsgefahr wird mittels der Vertiefung und dem dadurch gebildeten Schutz für den Katheter deutlich verringert. Das Gehäuse schützt in dieser Ausbildung den abgewinkelten Bereich des Katheter gegen mechanische Druckkräfte von außen.

Besonders bevorzugt verjüngt sich die Vertiefung zumindest von einer Seite her bis zu der Gehäuseöffnung allmählich und nach außen vorgewölbt. Diese Formgebung der Vertiefung ist insbesondere bei Verwendung eines flexiblen Katheters vorteilhaft, da ein Abkanten des Katheters sicher vermieden werden kann. Der Katheter kann jedoch in seinem abgewinkelten Bereich auch von einem festen Rohrstück gebildet werden.

Obgleich die Anordnung der Gehäuseöffnung für den Katheter in einer Vertiefung besonders vorteilhaft in Verbindung mit der erfindungsgemäßen Membrane und Membrane-Kanülen-Kombination ist, kann solch eine Ausbildung eines Gehäuses einer Konnektiervorrichtung, vorzugsweise einer Körperzugangsvorrichtung, auch vorteilhaft in Verbindung mit herkömmlichen Membranen und Membrane-Kanülen-Kombinationen vorgesehen sein. Die Anmelderin behält es sich vor, hierauf Ansprüche im Rahmen einer eigenen Anmeldung zu richten, insbesondere auf eine Körperzugangsvorrichtung nur mit den Merkmalen des Oberbegriffs von Anspruch 1 oder Anspruch 6.

Eine weitere Besonderheit einer erfindungsgemäßen Körperzugangsvorrichtung ist die kippsichere Abstützung der Fluidführungseinrichtung unmittelbar an einem Portkörper oder vorzugsweise an einem Gehäuse für die Membrane, das in dem Portkörper befestigbar ist. Auch auf diese besondere Ausbildung der mechanischen Verbindung der Fluidführungseinrichtung mit dem Portkörper können im Rahmen einer eigenen Anmeldung Ansprüche gerichtet werden, die nicht ausschließlich die erfindungsgemäße Membrane oder Membrane-Kanülen-Kombination betreffen.

Die Erfindung wird nachfolgend anhand eines bevorzugten Ausführungsbeispiels erläutert. Die anhand des Ausführungsbeispiels offenbar werdenden Merkmale bilden je einzeln und in jeder ihrer Merkmalskombinationen die Gegenstände der Ansprüche vorteilhaft weiter. Es zeigen:
- Figur 1: Eine erfindungsgemäße Membrane,
- Figur 2: eine erste Ausführungsform einer erfindungsgemäßen Konnektiervorrichtung in einem Längsschnitt,
- Figur 3: die Konnektiervorrichtung der Figur 2 in einer Ansicht,
- Figur 4: den Portkörper der Konnektiervorrichtung der Figuren 2 und 3,
- Figur 5: den Portkörper der Figur 4 mit der Membrane der Figur 1 in einem Längsschnitt und
- Figur 6: die Fluidführungseinrichtung der Figuren 2 und 3 in einem Längsschnitt.

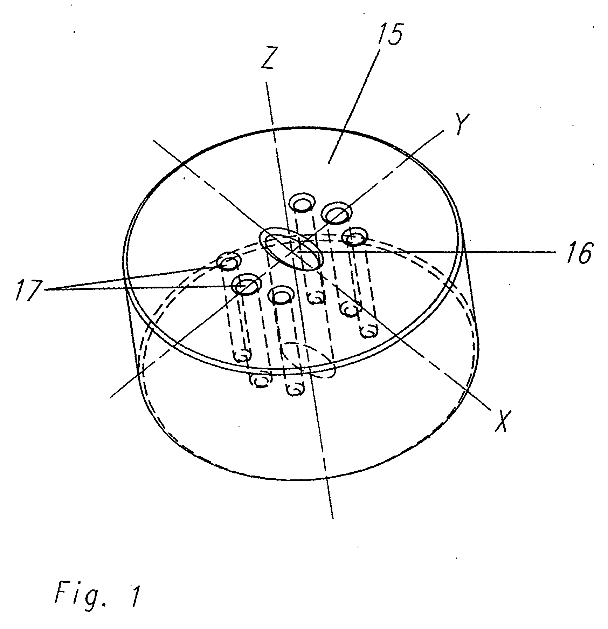

Figur 1 zeigt eine Membrane 15, die als Kreiszylinderkörper aus einem homogenen, elastischen Material, beispielsweise Silikon oder Latex, ausgebildet ist. Ein Durchgang 16 erstreckt sich konzentrisch zu einer zentralen Längsachse Z durch die Membrane 15 hindurch. Der Durchgang 16 ist geradzylindrisch und weist über seine gesamte Länge eine nach Größe und Form gleichbleibende Querschnittsfläche auf. Die Querschnittsfläche des Durchgangs 16 ist langgestreckt, d. h. sie weist eine lange Hauptachse X und eine kurze Hauptachse Y auf, die senkrecht zur Längsachse Z der Membrane 15 weisen. Im Ausführungsbeispiel ist die Querschnittsfläche oval, beispielsweise elliptisch.

Beabstandet von dem zentralen Durchgang 16 sind mehrere Hohlräume 17 in der Membrane 15 ausgebildet. Die Hohlräume 17 werden ebenfalls von einfach geradzylindrischen Durchgängen gebildet, die sich zwischen den beiden Stirnflächen der Membrane 15 durch die Membrane 15 hindurch erstrecken. Die Hohlräume 17 sind achssymmetrisch in bezug auf die lange Hauptachse X angeordnet. Sie sind ferner auch achssymmetrisch zu der kurzen Hauptachse Y angeordnet, wobei eine Häufung der Hohlräume 17 zu der kleinen Hauptachse Y hin auftritt. In der näheren Umgebung der langen Hauptachse X und insbesondere auf der langen Hauptachse X sind keine Hohlräume vorgesehen. Die Hohlräume 17 verlaufen parallel zu der Längsachse Z. Die Hohlräume 17 sind entlang einer Linie angeordnet, die näherungsweise oder vorzugsweise genau parallel beabstandet zu dem Rand des Durchgangs 16 um die Längsachse Z verläuft.

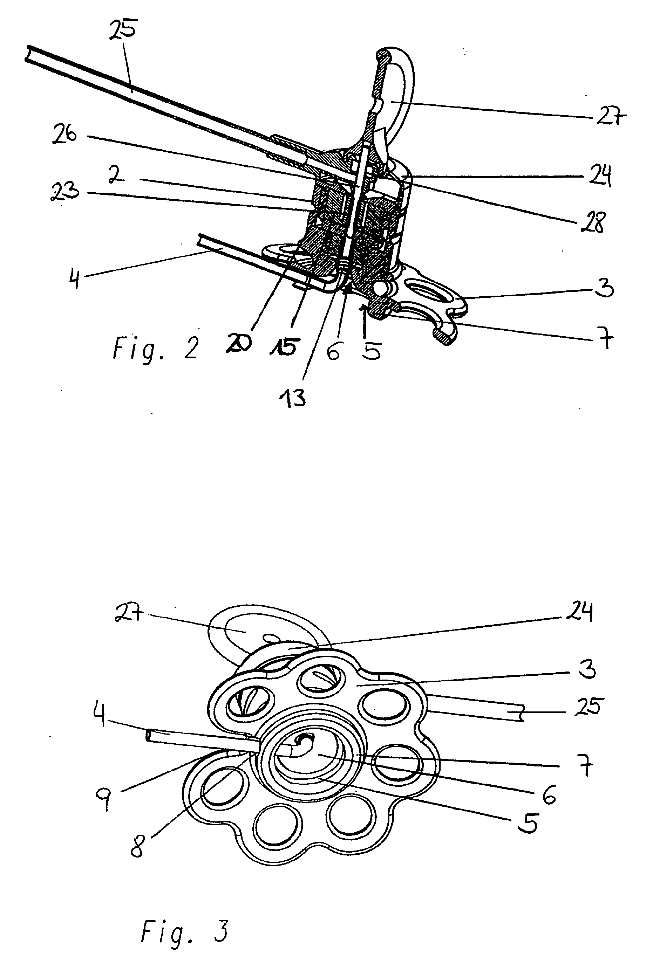

Figur 2 zeigt am Beispiel einer Konnektiervorrichtung, mit der zwei Katheter 4 und 25 miteinander verbunden werden, ein Einbaubeispiel für die Membrane 15. Die Membrane 15 ist in ein hohlzylindrisches, topfförmiges Membrangehäuse 20 eingesetzt. Das Membrangehäuse 20 fasst die Membrane 15 an deren Mantelfläche eng ein, d. h. die Membrane 15 wird über ihre gesamte äußere Mantelfläche radial von dem Membrangehäuse 20 abgestützt. Die Membrane 15 kann in dem Membrangehäuse 20 eingepresst und dadurch vorkomprimiert sein. An seiner Oberseite weist das Membrangehäuse 20 einen Boden mit einer zentralen Durchgangsöffnung auf.

Der Durchgang 16 der Membrane 15 wird von einer Kanüle 23 durchragt, die durch einen Boden des Membrangehäuses 20 eingeführt ist. Die Kanüle 23 ist ausreichend steif, um auch unter dem radialen Druck des elastischen Materials der komprimierten Membrane 15 einen Strömungsquerschnitt zur Durchleitung eines Fluids zu bilden. Die Membrane 15 wird zwischen der einfassenden Zylinderwandung des Membrangehäuses 20 und der durch den Durchgang 16 geführten Kanüle 23 elastisch in die Hohlräume 17 der Membrane 15 komprimiert. Obgleich es bevorzugt wird, dass die Hohlräume 17 durch die Kompression der Membrane 15 dicht geschlossen werden, ist solch ein dichter Verschluss der Hohlräume 17 nicht unumgänglich erforderlich, wenn ein Fluiddurchtritt durch die Hohlräume 17 anderweitig verhindert werden kann. Im Ausführungsbeispiel der Konnektiervorrichtung drückt die Membrane 15 mit ihren beiden Stirnflächen gegen Wandungen der Konnektiervorrichtung, nämlich gegen den Boden des Membrangehäuses 20 und gegen eine dem Boden gegenüberliegende Stirnfläche der Konnektiervorrichtung. Ein dichter Verschluss der Hohlräume 17 findet bereits gegen diese beiden Stirnflächen statt.

Die Kanüle 23 ist vorzugsweise geradzylindrisch mit einem Kreisquerschnitt. Der Außendurchmesser der Kanüle 13 ist größer als der in Richtung der kurzen Hauptachse Y gemessene Durchmesser der Membrane 15, wobei diese Angabe bezogen ist auf den in Figur 1 dargestellten unbelasteten Zustand der Membrane 15, in dem die Membrane 15 frei von äußeren Kräften ist. Bei der durch die Membrane 15 und die Kanüle 23 gebildeten Membrane-Kanülen-Kombination kann eine elastische Kompression der Membrane 15 allein durch die Aufweitung des Durchgangs 16 auf Grund der eingeführten Kanüle 23 herbeigeführt werden. Falls die Membrane 15 bereits vorkomprimiert, vorzugsweise radial vorkomprimiert, in dem Membrangehäuse 20 sitzt, kann der Durchmesser der Kanüle 23 auch kleiner sein als der in Richtung der kurzen Hauptachse Y gemessene Durchmesser des Durchgangs 16. Die Membrane 15 wird durch die eingeführte Kanüle 23 in diesem Falle lediglich zusätzlich zu ihrer Vorkompression noch stärker komprimiert. Der in Richtung der langen Hauptachse X gemessene Durchmesser des Durchgangs 16 ist größer als der Außendurchmesser der Kanüle 23.

Die Konnektiervorrichtung der Figur 2 ist eine Körperzugangsvorrichtung. Körperzugangsvorrichtungen stellen bevorzugte Ausführungsbeispiele einer erfindungsgemäßen Konnektiervorrichtung dar. Erfindungsgemäße Konnektiervorrichtungen werden jedoch nicht unumgänglich notwendig durch Körperzugangsvorrichtungen gebildet. Durch die nachfolgende Verwendung des Begriffs Körperzugangsvorrichtung sollen andere Konnektiervorrichtungen, für die die Erfindung einsetzbar ist, daher nicht ausgeschlossen werden.

Figur 3 zeigt die Körperzugangsvorrichtung der Figur 2 in einer Ansicht auf eine Unterseite.

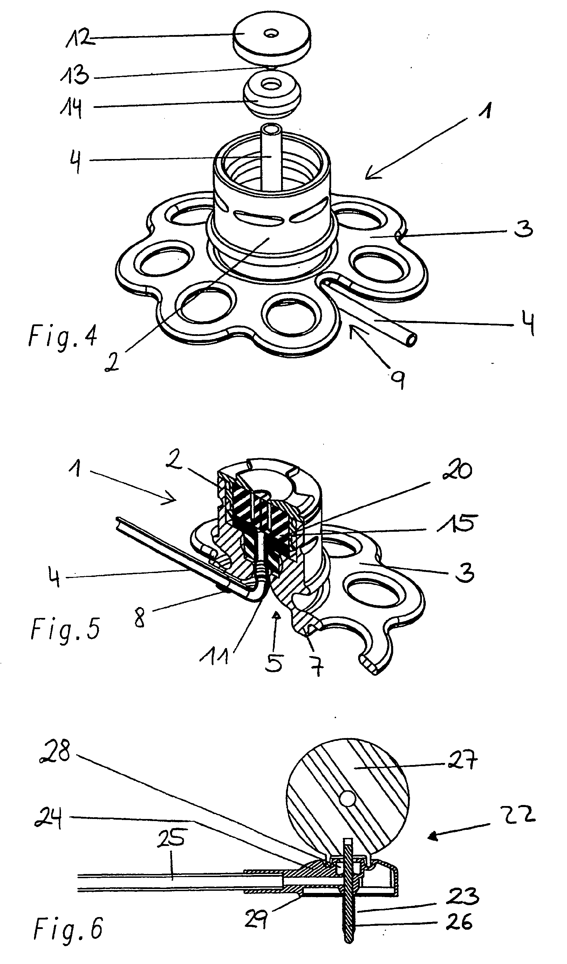

Die Körperzugangsvorrichtung wird gebildet von einem Portkörper 1 und einer Fluidführungseinrichtung 22, die einzeln in den Figuren 5 und 6 dargestellt sind. Die Figuren 2 und 3 zeigen den Portkörper 1 und die Fluidführungseinrichtung 22 im verbundenen Zustand, in dem durch die Vorrichtung und insbesondere durch die Membrane-Kanülen-Kombination 15,23 eine dichte Verbindung zwischen dem Katheter 4 und dem Katheter 25 hergestellt ist.

Der Portkörper 1 ist für eine perkutane Implantation im menschlichen Körper vorgesehen. Zur Verankerung im Zellgewebe, insbesondere unter der Haut, dient ein schirmförmig gewölbter, flächenhafter Verankerungskörper 3, von dem ein hohlzylindrisches Gehäuse 2 senkrecht aufragt. Das Gehäuse 2 ist an einer von dem Verankerungskörper 3 abgewandten Stirnseite offen. Das Gehäuse 2 und der Verankerungskörper 3 sind aus einem biokompatiblen Kunststoffmaterial einstückig gespritzt.

Der Katheter 4 ist im menschlichen Körper implantiert. Der Katheter 4 ragt mit einem Ende durch eine Öffnung, die im Bereich eines Bodens des Gehäuses 2 gebildet ist, in das Gehäuse 2 hinein. Der Boden wird durch einen Ringsteg 11 (Figur 5) auf der Höhe des Verankerungskörpers 3 bzw. im Bereich des Übergangs von dem Verankerungskörper 3 zu dem Gehäuse 2 gebildet.

Figur 4 zeigt, wie der Katheter 4 in dem Gehäuse 2 befestigt wird. Das Katheterende wird durch die Öffnung in das Gehäuse 2 eingeführt. Anschließend wird das Katheterende in einen Klemmring 14 eingeführt, und es wird ein Stutzen 13, der von einer scheibenförmigen Stützhülse 12 abragt und an seinem Außenmantel wulstförmige Ringe aufweist, in das von dem dem Klemmring 14 umgebende Katheterende hineingepresst. Im montierten Zustand, wie er in Figur 5 dargestellt ist, wird der Klemmring 14 in einem sich an die Gehäuseöffnung anschließenden Bereich des Gehäuses 2 aufgenommen. Über dem Klemmring 14 ist das Gehäuse 2 radial verbreitert. Die Stützhülse 12 liegt auf einer durch die Verbreiterung in dem Gehäuse 2 gebildeten Ringschulter auf.

Nachdem der Katheter 4 auf diese Weise gegen ein Herausrutschen durch die an der Unterseite des Gehäuses 2 gebildete Öffnung gesichert ist, wird das Membrangehäuse 20 mit der darin aufgenommenen Membrane 15 in das Gehäuse 2 so eingesetzt, dass die Membrane 15 mit ihrer Stirnfläche gegen eine Oberseite der Stützhülse 12 drückt und einen sich durch die Stützhülse 12 und deren Stutzen 13 erstreckenden Durchgang in den Katheter 4 rundherum abdichtet. Der Durchgang 16 der Membrane 15 liegt in der Flucht des Katheterendes. Das Gehäuse 2 und das Membrangehäuse 20 werden im Zuge des Einsetzens formschlüssig miteinander verbunden. Sie weisen zu diesem Zweck miteinander verrastende Rastmittel auf. Das Rastmittel des Gehäuses 2 wird durch eine vom Innenmantel des Gehäuses 2 radial vorragende Ringrippe gebildet, und das Membrangehäuse 20 ist an seinem Außenmantel mit einer entsprechenden Ringnut versehen, in die im eingesetzten Zustand des Membrangehäuses 20 der Ringsteg des Gehäuses 2 einrastet. Das Herstellen der Rastverbindung erzeugt ein gut hörbares Klickgeräusch. In Figur 5 ist der Portkörper 1 in diesem Zustand, d. h. nach Herstellung der Rastverbindung zwischen dem Portkörpergehäuse 2 und dem Membrangehäuse 20 dargestellt.

Wie in Figur 6 dargestellt, weist eine mit dem Portkörper 1 der Figur 5 formschlüssig verbindbare Fluidführungseinrichtung 22 ein Kanülengehäuse 24 auf, von dem die Kanüle 23 vorragt und in das der Katheter 25 quer zu der Kanüle 23 hineingeführt ist. Der Katheter 25 sitzt dicht in einem seitlichen Stutzen des Kanülengehäuses 24. Ein den Katheter 25 in dem Kanülengehäuse 24 verlängernder Fluidkanal führt in die Kanüle 23.

Zum Einführen der Kanüle 23 in den Durchgang 16 der Membrane 15 wird die Kanüle 23 von einem Konnektierstift 26 durchragt. Der Konnektierstift 26 dient nur dem Zweck, die Einführung der Kanüle 23 ohne Beschädigung der Membrane 15 sicherzustellen. Um dies zu erleichtern, ist ein vorderes Ende des Konnektierstifts 26 gerundet ausgebildet. Das vordere Ende des Konnektierstifts 26 ragt ein kleines Stück weit aus der Kanüle 23 hervor. In Verbindung mit dem Konnektierstift 26 wird ein weich gerundeter vorderer Abschluss für die Kanüle 23 erhalten. Das vordere Ende der Kanüle 23 kann auf diese Weise sogar scharfkantig sein.

Die Kanüle 23 erstreckt sich in dem Kanülengehäuse 24 von ihrem vorderen offenen Ende her gesehen über den Fluidkanal für den Katheter 25 hinaus. In dem verlängerten Bereich ist ein Septum 28 angeordnet. Der Konnektierstift 26 ist durch das Septum 28 in die Kanüle 23 eingeführt. Das Septum 28 stellt eine hermetische Abdichtung der Kanüle 23 sicher, wenn der Konnektierstift 26 eingeführt ist und auch nach einem Herausziehen des Konnektierstifts 26.

Das Kanülengehäuse 24 weist an einer Unterseite, über die die Kanüle 23 hinaus vorragt, Rastmittel 29 in Form von Rastrippen auf, die von einem umlaufenden, freien Rand des Kanülengehäuses 24 radial einwärts vorragen. Das Membrangehäuse 20 ist in einem von dem Verankerungskörper 3 abgewandten oberen Bereich mit entsprechenden Rastmitteln in Form von Einkerbungen versehen, in welche die Rastmittel 29 des Kanülengehäuses 24 einrasten. Im verrasteten Zustand umfasst das Kanülengehäuse 24 den oberen Öffnungsrand des Membrangehäuses 20 eng, wodurch eine kippsichere Führung der Fluidführungseinrichtung 22 an dem Membrangehäuse 20 und deshalb letztlich an dem Gehäuse 2 erhalten wird.

Um nach dem Verrasten des Portkörpers 1 und der Fluidführungseinrichtung 22 die Fluidverbindung zwischen dem Katheter 25 und dem Katheter 4 herzustellen, wird der Konnektierstift 26 aus der Kanüle 23 herausgezogen. Das Herausziehen wird durch die Ausbildung eines Konnektiergriffs 27 an einem aus dem Kanülengehäuse 24 herausragenden Ende des Konnektierstifts 26 erleichtert.

Eine Besonderheit der Körperzugangsvorrichtung ist die Anordnung der Öffnung für den Katheter 4 in einer Vertiefung 5 an der Unterseite des Portkörpers 1. Die Vertiefung 5 ist taschenförmig und wird von einem Begrenzungsring 7 umringt, der an der Unterseite des Portkörpers 1 etwa in Verlängerung des zylindrischen Gehäuses 2 von dem Verankerungskörper 3 vorragt. Innerhalb des Begrenzungsrings 7 verjüngt sich die Vertiefung 5 trichterförmig bis in die in dem Ringsteg 11 gebildete Gehäuseöffnung.

Im Verlauf der Verjüngung ist die Vertiefung 5 bis zu dem in das Gehäuse 2 führenden Öffnungsrand des Ringstegs 11 vorgewölbt weich gerundet, wodurch ein trompetenförmiger Trichter erhalten wird. Der Katheder 4 weist von der Gehäuseöffnung aus eine an die Form des Trichters angepasste Krümmung um etwa 90° auf. Der Trichter weist in der Vertiefung 5 eine senkrecht zum Öffnungsquerschnitt gemessene Tiefe auf, die ausreichend groß ist, um den Katheder 4 zumindest knickfrei um den erforderlichen Winkel, vorzugsweise 90°, umzulenken.

Unmittelbar im Anschluss an seinen gekrümmten bzw. umgelenkten Bereich ist der Katheder 4 durch einen in dem Begrenzungsring 7 ausgebildeten Führungsdurchgang 8 geführt. Der Führungsdurchgang 8 kann durch eine zur Unterseite hin offene Einkerbung in dem Begrenzungsring 7 gebildet werden. Vorzugsweise wird er jedoch wie im Ausführungsbeispiel durch ein Loch in dem Begrenzungsring 7 gebildet, durch das der Katheder 4 hindurchgeführt wird. Auf diese Weise wird der Katheder 4 gleichzeitig auch an der Unterseite der Körperzugangsvorrichtung fixiert.

Von der Gehäuseöffnung für den Katheder 4 aus in Verlängerung des Führungsdurchgangs 8 gesehen, weist der Verankerungskörper 3 eine Ausnehmung 9 auf, die sich vom äußeren Umfang her in den Verankerungskörper 3 erstreckt und so den Verankerungskörper 3 vom Rand her unterbricht. Der Katheder 4 wird im Bereich der Ausnehmung 9 aus dem Bereich des Verankerungskörpers 3 geführt. Durch die Ausnehmung 9 wird sichergestellt, dass der Verankerungskörper 3 nicht auf den Katheder 4 drücken und dadurch den Strömungsquerschnitt des Katheders 4 verringern oder den Katheder 4 gar gänzlich abklemmen kann. Desweiteren ermöglicht die Ausnehmung 9 eine besonders enge Führung des Katheders 4 am Portkörper 1.

### Bezugszeichen:

- 1: Portkörper
- 2: Gehäuse
- 3: Verankerungskörper
- 4: Katheder
- 4a: Kathederendstück
- 5: Vertiefung, Tasche
- 6: Trichter
- 7: Begrenzungsrippe
- 8: Führungsdurchgang
- 9: Ausnehmung
- 10: Verbindungsstück
- 10a: Klemmhülse
- 11: Ringsteg
- 12: Stützhülse
- 13: Stutzen
- 14: Klemmring
- 15: Membrane
- 16: Durchgang
- 17: Hohlraum
- 18: O-Ring
- 19: --
- 20: Membrangehäuse
- 21: Rastmittel
- 22: Fluidführungseinrichtung
- 23: Kanüle
- 24: Kanülengehäuse
- 25: Katheder
- 26: Konnektierstift
- 27: Konnektiergriff
- 28: Septum
- 29: Rastmittel
- X: lange Hauptachse
- Y: kurze Hauptachse
- Z: Längsachse

## Patentansprüche

1. Membrane-Kanulen-Kombination für biologische Anwendungen, die Kombination umfassend:
a) eine Kanüle (23) zur Durchleitung eines Fluids,
b) ein Gehäuse
c) und eine von dem Gehäuse aufgenommene Membrane (15) mit einem elastischen Membranmaterial, durch das ein Durchgang (16) gebildet ist, in den die Kanüle (23) eingeführt werden kann,
d) wobei der Durchgang (16) durch Einführung der Kanüle (23) aufgeweitet wird und das Membranmaterial quer zu dem Durchgang (16) gegen das Gehäuse und elastisch gegen die eingeführte Kanüle (23) drückt, so dass das Membranmaterial die Kanüle (23) dicht umgibt,
e) wobei die Membrane (15) quer zu dem Durchgang (16) kompressibel ist
f) und wobei der Durchgang (16) eine Querschnittsfläche mit einer langen Hauptachse (X) und einer zu der langen Hauptachse (X) senkrechten, kurzen Hauptachse (Y) aufweist,
**dadurch gekennzeichnet, dass**
g) die Länge der langen Hauptachse (X) der Querschnittsfläche des Durchgangs (16) größer als der Kanülendurchmesser ist, so dass der Durchgang (16) einen Hohlraum bildet, in den bei Einführung der Kanüle (23) das elastische Membranmaterial ausweichen kann.

2. Membrane-Kanülen-Kombination nach dem vorhergehenden Anspruch, **dadurch gekennzeichnet, dass** in dem elastischen Material der Membrane (15) wenigstens ein Hohlraum (17) ausgebildet ist, in den bei einer Kompression quer zu dem Durchgang (16) das elastische Material ausweichen kann.

3. Membrane-Kanülen-Kombination nach dem vorhergehenden Anspruch, **dadurch gekennzeichnet, dass** der wenigstens eine Hohlraum (17) neben dem Durchgang (16) angeordnet ist und sich vorzugsweise neben dem Durchgang (16) durch das elastische Material erstreckt.

4. Membrane-Kanülen-Kombination nach Anspruch 2 oder 3, **dadurch gekennzeichnet, dass** in dem elastischen Material der Membrane (15) mehrere der Hohlräume (17) ausgebildet und achssymmetrisch in Bezug auf eine Symmetrieachse (X) angeordnet sind, die sich in einer Querschnittsebene des Durchgangs (16) erstreckt.

5. Membrane-Kanülen-Kombination nach dem vorhergehenden Anspruch, **dadurch gekennzeichnet, dass** die Hohlräume (17) in Bezug auf eine zu der Querschnittsebene des Durchgangs (16) senkrechte Längsachse (Z) der Membrane (15) nicht rotationssymmetrisch angeordnet sind.

6. Membrane-Kanülen-Kombination nach einem der vorhergehenden Ansprüche, **dadurch gekennzeichnet, dass** die Querschnittsfläche der Membran oval ist

7. Membrane-Kanülen-Kombination nach einem der vorhergehenden Ansprüche, **dadurch gekennzeichnet, dass** die Querschnittsfläche der Membran einen Umfang mit einer stetigen Krümmung aufweist.

8. Membrane-Kanülen-Kombination gemäß einer Kombination von einem der Ansprüche 4 und 5 mit einem der Ansprüche 6 und 7, **dadurch gekennzeichnet, dass** die Hohlräume (17) achssymmetrisch zu beiden Seiten der langen Hauptachse (X) angeordnet sind.

9. Konnektiervorrichtung zur Verbindung einer Fluidführungseinrichtung (22) für ein biologisches oder biologisch wirksames Fluid mit einem Katheter (4), die Konnektiervorrichtung umfassend:
a) ein Gehäuse (2) mit einem Einlass für den Katheter (4),
b) eine Kanüle (23), die ein vorderes Ende der Fluidführungseinrichtung (22) bildet,
c) und eine elastische Membrane (15) mit einem Durchgang (16), in den die Kanüle (23) zur Herstellung der Verbindung einführbar ist,
d) wobei die Membrane (15) von dem Gehäuse (2) derart aufgenommen ist, dass durch die Membrane (15) eine dichte Verbindung zwischen dem Katheter (4) und der Kanüle (23) hergestellt wird, **dadurch gekennzeichnet, dass**
e) die Membrane-Kanülen-Kombination gemäß einem der vorhergehenden Ansprüche ausgebildet ist.

10. Konnektiervorrichtung nach dem vorhergehenden Anspruch, **dadurch gekennzeichnet, dass** die Konnektiervorrichtung eine Körperzugangsvorrichtung bildet, der Katheter (4) implantierbar und das Gehäuse (2) ein perkutan oder subkutan implantierbarer Portkörper (1) ist.

11. Konnektiervorrichtung nach einem der zwei vorhergehenden Ansprüche, **dadurch gekennzeichnet, dass** der Gehäuseeinlass für den Katheter (4) in einer Vertiefung (5) einer Gehäuseoberlläclae ausgebildet ist und die Vertiefung (5) eine für die Aufnahme eines gekrümmten Stücks des Katheters (4) ausreichende Größe aufweist.

12. Konnektiervorrichtung nach dem vorhergehenden Anspruch, **dadurch gekennzeichnet, dass** die Vertiefung (5) sich zu dem Einlass hin mit einer Wölbung nach außen trichterförmig allmählich verjüngt.

## Claims

1. A membrane-cannula combination for biological applications, said combination comprising:
a) a cannula (23) for conveying a fluid;
b) a casing;
c) and a membrane (15) accommodated by the casing and comprising an elastic membrane material through which a passage (16) is formed, into which the cannula (23) can be inserted;
d) wherein the passage (16) is expanded by inserting the cannula (23) and the membrane material presses against the casing, perpendicular to the passage (16), and elastically presses against the inserted cannula (23), such that the membrane material surrounds the cannula (23) in a seal,
e) wherein the membrane (15) can be compressed perpendicular to the passage (16),
f) and wherein the passage (16) exhibits a cross-sectional area comprising a long main axis (X) and a short main axis (Y) perpendicular to the long main axis (X),
**characterised in that**
g) the length of the long main axis (X) of the cross-sectional area of the passage (16) is larger than the cannula diameter, such that the passage (16) forms a hollow space into which the elastic material of the membrane can yield when the cannula (23) is inserted.

2. The membrane-cannula combination according to the preceding claim, **characterised in that** at least one hollow space (17) is formed in the elastic material of the membrane (15), and the elastic material can yield into the at least one hollow space (17) when compressed perpendicular to the passage (16).

3. The membrane-cannula combination according to the preceding claim, **characterised in that** the at least one hollow space (17) is arranged next to the passage (16) and preferably extends through the elastic material next to the passage (16).

4. The membrane-cannula combination according to claim 2 or 3, **characterised in that** a number of hollow spaces (17) are formed in the elastic material of the membrane (15) and are arranged axially symmetrical with respect to an axis of symmetry (X) which extends in a cross-sectional plane of the passage (16).

5. The membrane-cannula combination according to the preceding claim, **characterised in that** the hollow spaces (17) are not arranged rotationally symmetrical with respect to a longitudinal axis (Z) of the membrane (15) which is perpendicular to the cross-sectional plane of the passage (16).

6. The membrane-cannula combination according to any one of the preceding claims, **characterised in that** the cross-sectional area of the membrane is oval.

7. The membrane-cannula combination according to any one of the preceding claims, **characterised in that** the cross-sectional area of the membrane exhibits a circumference with a constant curvature.

8. The membrane-cannula combination in accordance with a combination of any one of claims 4 and 5 and any one of claims 6 and 7, **characterised in that** the hollow spaces (17) are arranged axially symmetrical on both sides of the long main axis (X).

9. A connecting device for connecting a fluid guiding means (22) for a biological or biologically active fluid to a catheter (4), said connecting device comprising:
a) a casing (2) comprising an inlet for the catheter (4);
b) a cannula (23) which forms a front end of the fluid guiding means (22);
c) and an elastic membrane (15) comprising a passage (16) into which the cannula (23) can be inserted, to establish the connection;
d) wherein the membrane (15) is accommodated by the casing (2) in such a way that a sealed connection is established between the catheter (4) and the cannula (23) by the membrane (15), **characterised in that**
e) the membrane-cannula combination is formed in accordance with any one of the preceding claims.

10. The connecting device according to the preceding claim, **characterised in that**: the connecting device forms a body access device; the catheter (4) can be implanted; and the casing (2) is a port body (1) which can be percutaneously or subcutaneously implanted.

11. The connecting device according to any one of the preceding two claims, **characterised in that** the casing inlet for the catheter (4) is formed in a recess (5) of a casing surface, and the recess (5) exhibits a sufficient size to accommodate a curved section of the catheter (4).

12. The connecting device according to the preceding claim, **characterised in that** the recess (5) gradually tapers towards the inlet, in the shape of a funnel, with an outward swell.

## Revendications

1. Combinaison membrane et canule pour des applications biologiques, la combinaison comprenant :
a) une canule (23) pour le passage d'un fluide,
b) un boîtier
c) et une membrane (15) logée par le boîtier avec un matériau de membrane élastique, dans lequel est constitué un passage (16) dans lequel la canule (23) peut être introduite,
d) le passage (16) étant élargi par l'introduction de la canule (23) et le matériau de membrane poussant transversalement au passage (16) contre le boîtier et élastiquement contre la canule (23) introduite de sorte que le matériau de membrane entoure de manière étanche la canule (23),
e) la membrane (15) étant compressible transversalement au passage (16),
f) et le passage (16) présentant une surface de section avec un axe principal (X) long et un axe principal (Y) court, perpendiculaire à l'axe principal long (X),
**caractérisée en ce que**
g) la longueur de l'axe principal (X) long de la surface de section du passage (16) est plus grande que le diamètre de la canule de sorte que le passage (16) forme un espace creux, dans lequel lors de l'introduction de la canule (23), le matériau de membrane élastique peut s'échapper.

2. Combinaison membrane et canule selon la revendication précédente, **caractérisée en ce que** dans le matériau élastique de la membrane (15) est réalisé au moins un espace creux (17), dans lequel lors d'une compression, le matériau élastique peut s'échapper transversalement au passage (16).

3. Combinaison membrane et canule selon la revendication précédente, **caractérisée en ce que** l'au moins un espace creux (17) est disposé à côté du passage (16) et s'étend de préférence à côté du passage (16) au travers du matériau élastique.

4. Combinaison membrane et canule selon la revendication 2 ou 3, **caractérisée en ce que** dans le matériau élastique de la membrane (15), plusieurs des espaces creux (17) sont réalisés et sont disposés de manière axisymétrique par rapport à un axe de symétrie (X) qui s'étend dans un plan de section du passage (16).

5. Combinaison membrane et canule selon la revendication précédente, **caractérisée en ce que** les espaces creux (17) ne sont pas disposés de manière symétrique en rotation par rapport à un axe longitudinal (Z) de la membrane (15) perpendiculaire au plan de section du passage (16).

6. Combinaison membrane et canule selon l'une quelconque des revendications précédentes, **caractérisée en ce que** la surface de section de la membrane est ovale.

7. Combinaison membrane et canule selon l'une quelconque des revendications précédentes, **caractérisée en ce que** la surface de section de la membrane présente une périphérie avec une courbure continue.

8. Combinaison membrane et canule selon une combinaison de l'une quelconque des revendications 4 et 5 avec l'une quelconque des revendications 6 et 7, **caractérisée en ce que** les espaces creux (17) sont disposés de manière axisymétrique des deux côtés du long axe principal (X).

9. Dispositif de connexion pour la liaison d'un dispositif de guidage de fluide (22) pour un fluide biologique ou actif biologiquement avec un cathéter (4), le dispositif de connexion comportant :
a) un boîtier (2) avec une admission pour le cathéter (4),
b) une canule (23) qui forme une extrémité avant du dispositif de guidage de fluide (22),
c) et une membrane élastique (15) avec un passage (16), dans lequel la canule (23) peut être introduite pour l'établissement de la liaison,
d) la membrane (15) étant logée par le boîtier (2) de telle sorte que par la membrane (15), une liaison étanche entre le cathéter (4) et la canule (23) soit établie, **caractérisé en ce que**
e) la combinaison membrane et canule est réalisée selon l'une quelconque des revendications précédentes.

10. Dispositif de connexion selon la revendication précédente, **caractérisé en ce que** le dispositif de connexion forme un dispositif d'accès au corps, le cathéter (4) peut être implanté et le boîtier (2) est un corps à port (1) pouvant être implanté en percutané ou sous-cutané.

11. Dispositif de connexion selon l'une quelconque des deux revendications précédentes, **caractérisé en ce que** l'admission de boîtier pour le cathéter (4) est réalisée dans une cavité (5) d'une surface de boîtier et la cavité (5) présente une grandeur suffisante pour le logement d'une pièce cintrée du cathéter (4).

12. Dispositif de connexion selon la revendication précédente, **caractérisé en ce que** la cavité (5) se rétrécit petit à petit en forme d'entonnoir vers l'admission avec une courbure vers l'extérieur.
